# EUROPEAN PATENT APPLICATION

(11) **EP 4 555 922 A1**
(43) Date of publication of application: **21.05.2025**
(21) Application number: 24203089.8
(22) Date of filing: 27.09.2024
(51) Int. Cl.: A61B 5/00, A61B 8/00, A61B 18/14, A61M 25/00, A61N 1/00, H05K 1/02

(54) **PCB CABLE ASSEMBLY FOR A CATHETER**

(30) Priority: 29.09.2023 US 202363541306 P; 03.09.2024 US 202418822984
(71) Applicant: Biosense Webster (Israel) Ltd., 2066717 Yokneam (IL)
(72) Inventor: GOVARI, Assaf, 2066717 Yokneam (IL); KATZIR, Stanislav, 2066717 Yokneam (IL); SITNITSKY, Ilya, 2066717 Yokneam (IL); SOBOL, Sergey, 2066717 Yokneam (IL); SHECHTMAN, Alexander, 2066717 Yokneam (IL)
(74) Representative: Carpmaels & Ransford LLP

(57) **Abstract**

A catheter with a distal end assembly, an elongate flexible PCB (Printed Circuit Board) being housed within a shaft of the catheter, the elongate flexible PCB extending substantially along a full length of the shaft and including a PCB distal portion including a plurality of electrical connection pads, a PCB proximal portion including a plurality of conductive traces corresponding to the plurality of electric connection pads of the PCB distal portion, a PCB median portion including a plurality of conductive traces laid longitudinally along more than 75% of a length of the elongate flexible PCB, the conductive traces continuing the conductive traces of the PCB proximal portion and electrically connected to the plurality of electrical pads of the PCB distal portion, and a plurality of slits cutting through the PCB median portion between the conductive traces and extending along the conductive traces. Related apparatus and methods are also described.

## Description

### RELATED APPLICATION/S

This application claims the benefit of priority under 35 USC 119(e) of U.S. Provisional Patent Application No. 63/541,306 filed 29 September 2023, the contents of which are incorporated herein by reference in their entirety.

### TECHNOLOGICAL FIELD

The present disclosure describes examples relating to a conductive cable and to a method for production of the conductive cable, and, more particularly, but not exclusively, to a conductive cable product which includes an elongate flexible Printed Circuit Board (PCB) cable along a length of which are drawn multiple conductive traces and through slits cut between the traces, thereby providing an elongate flexible PCB conductive cable.

### BACKGROUND

A catheter may be required to advance through tight areas with complex geometry. To enable reaching a desired location under such challenging conditions, the catheter should be very flexible. Today, wires running along a length of the catheter are used to provides electrical connections between the sensors and/or electrodes at a distal end of the catheter, and a connector and/or a plug at a proximal end of the catheter. The wires provide flexibility.

### OVERVIEW

A problem with using wires relates to practicality or manufacturability of a bundle of conductive wires. Electrically connecting each of the wires at each end of a catheter is a difficult manual process. The process is difficult due to the number of wires that need to be connected on a small footprint.

A solution to the above-mentioned problem includes replacing wires with a cable assembly formed from a flexible PCB strip that includes electrical connection pads at one or both of its ends, and conductive traces.

In some embodiments, the cable assembly may be about 1-2 meters long, e.g. 1.5 meters long, so that the cable assembly extends along a length of a catheter shaft.

A further potential improvement is related to increasing flexibility when multiple conductors are extended along a catheter, by making through cuts along a length of a flexible PCB between conductive traces, to improve flexibility of the PCB along a shaft or lumen of a catheter, and produce a more-flexible PCB cable assembly. Such through cuts potentially improve the flexibility of the PCB, thereby potentially improving the flexibility of the PCB cable assembly and of the catheter, and enable replacing multiple wire leads with the flexible PCB. The flexible PCB after through cutting is potentially more amenable to rolling or twisting the PCB to be a compact bundle of conductive traces as a PCB cable assembly which is threaded through a catheter and/or a lumen of the catheter.

Having multiple traces on the flexible PCB produces an equivalent of a cable with multiple wires.

In some embodiments, the traces on the flexible PCB may optionally be shielded from electromagnetic (EM) interference, by using EM shielding integrated in the flexible PCB itself and/or by providing EM shielding to the traces themselves.

In some embodiments, the traces on the flexible PCB may optionally be shielded from interference, by twisting the traces around each other, in a manner similar to a twisted pair of wires.

In some embodiments, the traces on the flexible PCB may optionally end with connectors and/or connection pads at one or both ends.

Another non-limiting example method for reducing the cost and/or speed of manufacturing the multiple conductors includes producing multiple traces on a flexible PCB to be used instead of threading multiple wires through a catheter, or even threading multiple wires through multiple lumens in a catheter, thereby potentially reducing cost and/or speed of manufacturing a device which uses such multiple conductors.

Another non-limiting example method for reducing the cost and/or speed of manufacturing the PCB cable assembly includes producing contacts at one or both ends of the conductors, making it easy to connect to the conductors. In some embodiments, the through cuts extend along a length of the flexible PCB between one or both ends of the flexible PCB and do not extend all the way to the end, thereby maintaining an end with a controlled spacing between the conductors, which enable producing a connector to connect to multiple conductors at once, thereby potentially reducing cost and/or speed of manufacturing a device which uses such multiple conductors.

Another non-limiting example method for reducing the cost and/or speed of manufacturing a device using the PCB cable assembly includes producing sensors upon the PCB, eliminating a need to attach multiple sensors, each one to one or more corresponding conductor(s), thereby potentially reducing cost and/or speed of manufacturing a device which uses such multiple sensors.

### BRIEF DESCRIPTION OF THE DRAWING(S)

Some examples of the disclosure are herein described, by way of example only, with reference to the accompanying drawings. With specific reference now to the drawings in detail, it is stressed that the particulars shown are by way of example and for purposes of illustrative discussion of embodiments of the disclosure. In this regard, the description taken with the drawings makes apparent to those skilled in the art how embodiments of the disclosure may be practiced.

In the drawings:
Figure 1A is an illustration of an example catheter-based electrophysiology mapping and ablation system.
Figure 1B is a partly exploded view of an example distal end assembly of a non-limiting example of a catheter-based electrophysiology device;
Figure 2A is a simplified block diagram illustration of a PCB cable assembly for connecting a distal end assembly to a proximal end connector according to an example embodiment;
Figure 2B is a simplified illustration of is a simplified illustration of a PCB cable assembly according to an example embodiment;
Figure 3 is a simplified illustration of an elongate flexible PCB according to an example embodiment;
Figure 4A is a simplified illustration of a PCB cable assembly according to an example embodiment;
Figure 4B is a simplified illustration of an elongate flexible PCB according to an example embodiment; and
Figure 5 is a simplified flow chart illustration of a method of connecting a distal end assembly of a catheter to a connector at a proximal end of the catheter, according to an example embodiment.

### DETAILED DESCRIPTION

The present disclosure describes examples relating to a PCB cable assembly and to a method for production of the PCB cable assembly, and, more particularly, but not exclusively, to a conductive cable product which includes an elongate flexible Printed Circuit Board (PCB) cable along a length of which are drawn multiple conductive traces and through slits cut between the traces, thereby providing an elongate flexible PCB conductive cable and, more particularly, but not exclusively, to a PCB cable assembly within a catheter.

### Introduction

A catheter may be required to advance through tight areas with complex geometry. To enable reaching a desired location under such challenging conditions, the catheter should be very flexible. Today, wires running through lumens along a length of the catheter are used to provide electrical connections between the sensors and/or electrodes at a distal end of the catheter, and a connector and/or a plug at a proximal end of the catheter. The wires provide flexibility.

Manufacturing a plurality of conductors along a length of a catheter by using a plurality of wires requires a significant amount of manual labor. Manufacturing and assembly of the conductors in the catheter is potentially simplified by using a flexible PCB upon which are arranged a required number of conductive traces.

A typical state of the art flexible PCB does not provide the flexibility needed for negotiating bending angles which a catheter may need to negotiate.

However, cutting multiple slits through an elongate flexible PCB, parallel to and between multiple traces laid along the elongate flexible PCB produces multiple narrower flexible PCB strips, thereby producing a PCB cable assembly which is potentially more flexible than an uncut flexible PCB.

Cutting the slits enables an optional additional action - twisting the narrower flexible PCB strips, potentially making the PCB cable assembly have a small diameter than a width of the uncut flexible PCB, potentially able to fit into a narrower catheter.

Cutting the slits enables another optional additional action - rolling the narrower flexible PCB strips, potentially making the PCB cable assembly have a small diameter than a width of the uncut flexible PCB, potentially able to fit into a narrower catheter.

### Remarks about a number of traces per flexible PCB, and a number of flexible PCBs in a catheter

In some embodiments, one or more traces may be placed on a flexible PCB, by way of some non-limiting examples, 1, 2, 3, 4, 5, and up to 10, 15 or even 20 traces.

In some embodiments, more than one flexible PCB may be inserted into a catheter, by way of some non-limiting examples, 1, 2, 3, 4, 5, and up to 10, 15 or even 20 flexible PCBs.

### Remarks about length of slits

In some embodiments, the slits may extend most, but not all, of a length of the elongate flexible PCB.

For example, all the slits may extend 75%, 80%, 85%, 90% 95% or even up to 98% or 99% of a length of the elongate flexible PCB, yet not reach either end of the elongate flexible PCB. Proximal and distal ends of the elongate flexible PCB are not cut through, and the remaining material connects the separated-by-the-slits strips, making them potentially easier to handle as a group of strips.

For example, connector pads may be structed at a proximal and/or a distal end of the elongate flexible PCB. Such connector pads are potentially easy to connect to, because their spacing is known despite any twists and turns the elongate flexible PCB may take along its length.

In some embodiments, one or more of the slits may extend until an end of the elongate flexible PCB.

In an embodiment where one slit extends all the way to an end of the elongate flexible PCB, the slit separates the elongate flexible PCB to two strips, attached at one end and separated at another end. The two strips can now optionally be twisted about each other, producing a twisted cable, if so desired. It is noted that in an embodiment where one slit extends all the way to both ends of the elongate flexible PCB, two separate strips are produced, and if so desired, the two separate strips can be twisted about each other, producing a twisted cable.

In some embodiments more than one slit extends all the way to an end of the elongate flexible PCB, and the slits separate the elongate flexible PCB to several strips, attached at one end and separated at another end. One or more of the several strips can now optionally be twisted about each other, producing a twisted cable, if so desired. It is noted that in an embodiment where more than one slit extends all the way to both ends of the elongate flexible PCB, several separate strips are produced, and if so desired, two or more of the several separate strips can be twisted about each other, producing a twisted PCB cable assembly.

In some embodiments, when it is desired to produce at least one twisted pair of conductors, one can optionally produce slits on either side of two neighboring conductive traces, the slits extending all the way to at least one end of the elongate flexible PCB, producing two neighboring conductive traces free at at least one end, and optionally twist the two conductive traces about each other, producing a twisted pair.

In some embodiments, when it is desired to produce at least one twisted group of conductors, one can optionally produce slits on either side of a group of neighboring conductive traces, the slits extending all the way to at least one end of the elongate flexible PCB, producing a group of neighboring conductive traces free at at least one end, and optionally twist the group conductive traces about each other, producing a twisted group. A number of traces in the group may include, by way of some non-limiting examples, 2, 3, 4, 5, up to 10, 15 or 20 traces.]

### Remarks about length of the PCB cable assembly

One purpose of conductive traces on the elongate flexible PCB is to connect between a device used inside a patient's body, by way of a non-limiting example a heart-related device at a distal end of a catheter such as a heart procedure catheter, and instruments at a proximal end of the catheter, outside the patient's body.

In some embodiments, the elongate flexible PCB may extend most, but not all, of a length of a catheter, for example 50%, 60%, 70%, 80%, 90%, 95% and even 98% or 99% of the length of the catheter. In some embodiments, the elongate flexible PCB may extend all of a length of a catheter. In some embodiments, the elongate flexible PCB may extend more than a length of a catheter.

### Remarks about proximal and distal ends of the PCB cable assembly

In some embodiments, a proximal end of the PCB cable assembly includes pads for electrical connection to an electrical connector, to be used as a connection to connect various electrical devices outside a catheter and outside a patient's body.

In some embodiments, one end of the PCB cable assembly may optionally include pads to connect to a connection plug at an end of a catheter, for example a proximal end or a distal end.

In some embodiments, one end of the PCB cable assembly may optionally include pads to connect to an end assembly comprising electrodes and/or sensors, and/or another PCB.

In some embodiments, a distal end of the elongate flexible PCB includes pads for electrical connection to an electrical connector, to be used as a connection to connect various sensors, electrodes and/or additional electrical devices outside a catheter and within a patient's body.

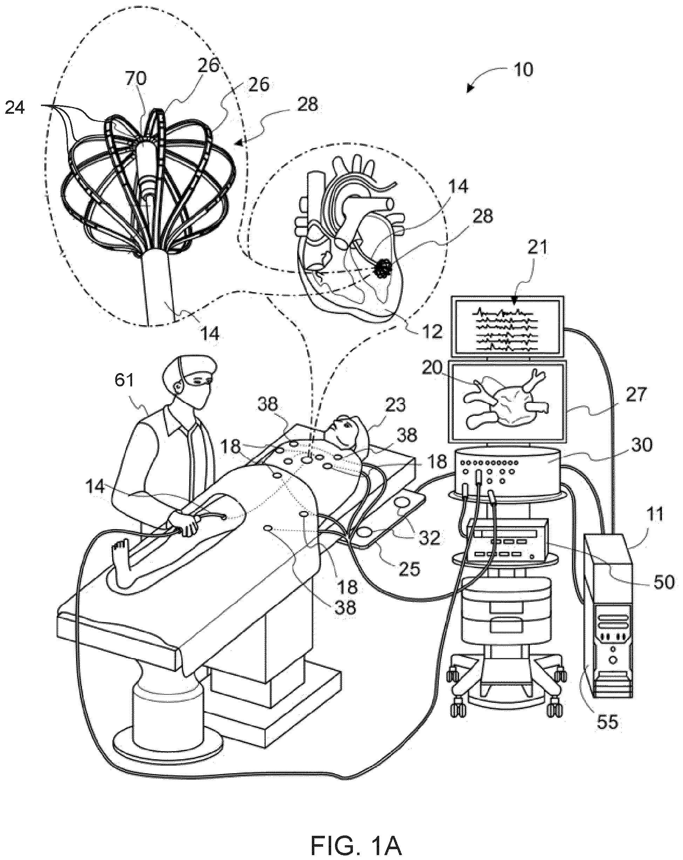

Reference is made to Figure 1A, which is an illustration of an example catheter-based electrophysiology mapping and ablation system 10. System 10 includes multiple catheters, which are percutaneously inserted by a physician 61 through the patient's vascular system into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An example catheter 14 that is configured for ablating tissue and/or for sensing electrical cardiac activity and/or mapping is illustrated herein.

Catheter 14 is an exemplary catheter having an end effector at its distal tip 28 that includes an expandable assembly, having one and preferably multiple electrodes 26 optionally distributed over a plurality of flexible spline elements 24. The electrodes 26 are generally configured for delivering ablation energy to tissue and/or for sensing cardiac electrical signals. Catheter 14 additionally includes one or more position sensors 70 embedded in or near distal tip 28 for tracking position and orientation of distal tip 28. Optionally and preferably, position sensor 70 is a magnetic based position sensor, for example a position sensor including three magnetic coils for sensing three-dimensional (3D) position and orientation; or a position sensor including one magnetic coil, for sensing a single direction.

Each of the magnetic based position sensors 70 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 70. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

Physician 61 may place a distal tip 28 of catheter 14 in contact with the heart wall for sensing a target site in heart 12. For ablation, physician 61 may similarly place a distal end of an ablation catheter in contact with a target site for ablating tissue.

System 10 includes one or more electrode patches 38 positioned for skin contact on patient 23 to establish location reference for location pad 25 as well as impedance-based tracking of electrodes 26. For impedance-based tracking, electrical current is directed to electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 records and displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software stored therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

In some examples, an irrigation module is provided for delivering irrigation fluid, such as saline solution, to the location of treatment. The irrigation module may comprise a pump and an associated fluid tank.

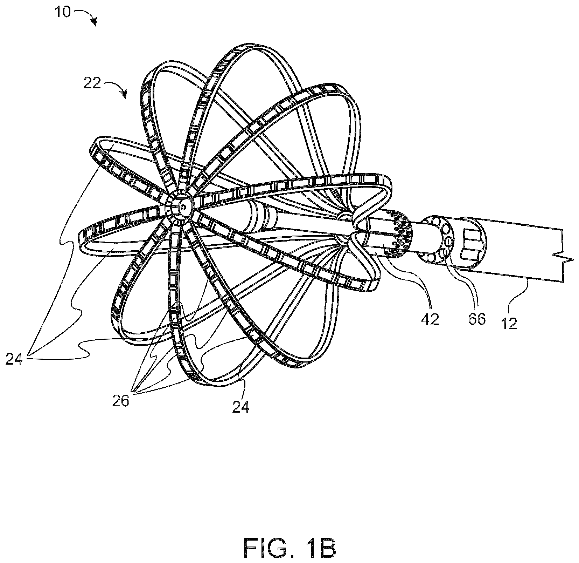

For purpose of better understanding some embodiments of the present disclosure, reference is first made to the construction of a non-limiting example of a catheter-based electrophysiology device as illustrated in Figure 1B.

Figure 1B is a partly exploded view of an example distal end assembly of a non-limiting example of a catheter-based electrophysiology device, in the non-limiting example of Figure 1B, a configuration called a basket catheter.

The non-limiting example shown in Figure 1B is a distal end assembly of a catheter, for operation within a patient's body.

In the example an end assembly 10 of a catheter 12 has a basket shape with a plurality of splines 24.

In some embodiment, a spline 24 may optionally include one or more electrodes 26 and/or one or more sensors 26. In some embodiments the electrodes 26 and/or sensors 26 may be mounted on a flexible PCB included in the spline 24.

In some embodiments ends 42 (only some of which are labeled, for the sake of simplicity) of the splines 24 are optionally provided with electrical connection to wires (not shown) passing through lumens 66 in the catheter 12.

As mentioned above, manufacturing a catheter which has plurality of conductors passing through lumens along a length of the catheter by threading the wires through the lumens requires a significant amount of manual labor. Manufacturing and assembly of the conductors in the catheter is potentially simplified by using a flexible PCB upon which are arranged a required number of conductive traces.

Also noted is that multiple wires threaded through multiple lumens in a catheter may not provide the flexibility needed for negotiating bending angles which a catheter may need to negotiate.

In various descriptions below, embodiments are described which are suitable, by way of some non-limiting examples, for electrically connecting sensors for operation within a heart, through a catheter, to instruments outside a patient's body.

An aspect of some embodiments includes replacing the wires (not shown) passing through lumens 66 in the catheter 12, with an elongate flexible PCB including multiple conductive traces serving for signal conductance along the catheter.

Before explaining at least one embodiment of the disclosure in detail, it is to be understood that the disclosure is not necessarily limited in its application to the details of construction and the arrangement of the components and/or methods set forth in the following description and/or illustrated in the drawings. The disclosure is capable of other embodiments or of being practiced or carried out in various ways.

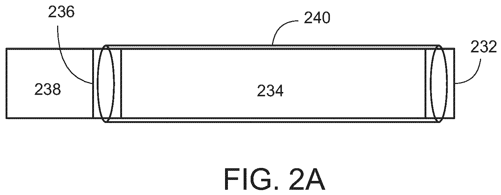

Reference is now made to Figure 2A, which is a simplified block diagram illustration of a PCB cable assembly for connecting a distal end assembly to a proximal end connector according to an example embodiment.

Figure 2A is intended to illustrate a concept of an example elongate flexible PCB 234, within a catheter 240.

In some embodiments the elongate flexible PCB 234 has a distal end connector 236, with electrical connection(s) for connecting to a distal assembly 238, by way of a non-limiting example, to a catheter-based electrophysiology device, optionally the non-limiting example of Figure 1B, a configuration called a basket catheter sensor.

In some embodiments the elongate flexible PCB 234 has a proximal end connector 232, with electrical connection(s) for connecting to, by way of a non-limiting example, medical instruments to use signal|(s) from the distal assembly 238.

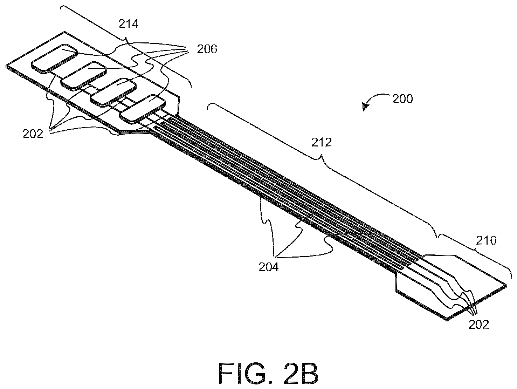

Reference is now made to Figure 2B, which is a simplified illustration of a PCB cable assembly according to an example embodiment.

Figure 2B shows an elongate flexible PCB 200, having multiple conductive traces 202 laid along a length of the elongate flexible PCB 200, and multiple slits 204 cutting through the elongate flexible PCB 200 parallel to and between the conductive traces 202.

Figure 2B shows three sections of the elongate flexible PCB 200 - a proximal end section 210, a mid-section 212, and a distal end section 214.

In some embodiments the end section 210 may be located at a proximal end section of a catheter, the mid-section 212 may pass through a shaft of the catheter, and the end section 214 may be located at a distal end section of the catheter.

In some embodiments the end section 210 may be located at a distal end section of a catheter, the mid-section 212 may pass through a shaft of the catheter, and the end section 214 may be located at a proximal end section of the catheter.

In some embodiments, the mid-section 212 extends 75%, 80%, 85%, 90% 95% or even up to 98%, or 99% of a length of the elongate flexible PCB 200, yet not reach either end of the elongate flexible PCB. The proximal end 210 and the distal end 214 of the elongate flexible PCB 200 are not cut through, and the remaining material connects the separated-by-the-slits strips, making them potentially easier to handle as a group of strips.

In some embodiments, the elongate flexible PCB 200 is sized to be packaged within a catheter.

In some embodiments, a length of the elongate flexible PCB 200 is designed to extend a full length of a catheter (not shown) into which the elongate flexible PCB 200 is planned to be inserted.

In some embodiments, a length of the elongate flexible PCB 200 is designed to extend more than a full length of a catheter (not shown) into which the elongate flexible PCB 200 is planned to be inserted, so the some of the proximal end section 210 and/or the distal end section 214 may jut out of the catheter.

In some embodiments, some or all of the proximal end section 210 is at an end of a catheter designed to be the end remaining outside of a patient's body.

In some embodiments, the conductive traces 202 are optionally electrically connected to electric components 206 such as electrodes 206 or sensors 206. Figure 2B shows the electric components at the distal end section 214. However, such electric components may be attached to the conductive traces 202 at the proximal end section 210.

In some embodiments, the conductive traces 202 are optionally electrically connected to electric connection pads (not shown), for connecting to additional circuitry.

Figure 2B shows the electric components 206 at the distal end section 214, so electric connection pads may be at the proximal end section 210. However, such electric connection pads may also optionally be at the distal end section 214.

In some embodiments, a width of the elongate flexible PCB 200 is designed to be less than a diameter of a catheter (not shown) into which the elongate flexible PCB 200 is planned to be inserted.

In some embodiments, a width of the elongate flexible PCB 200 is designed to be less than a circumference of a catheter (not shown) into which the elongate flexible PCB 200 is planned to be inserted, and the elongate flexible PCB 200 is inserted with the catheter and lies along the circumference of the catheter against an inner surface of the catheter.

In some embodiments, the elongate flexible PCB 200 is designed to be twisted and/or rolled. When twisted or rolled, an outer diameter of the elongate flexible PCB 200 is less than a width of the elongate flexible PCB 200, and can be inserted into a catheter diameter smaller than the width of the elongate flexible PCB 200.

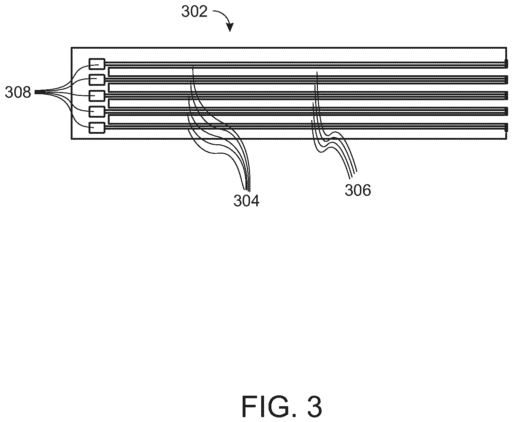

Reference is now made to Figure 3, which is a simplified illustration of an elongate flexible PCB according to an example embodiment.

Figure 3 is intended to show an embodiment where an elongate flexible PCB, forming a PCB cable assembly, includes slits and conductive traces similarly to slits 204 and conductive traces 202 shown in the mid-section 212 of Figure 2B.

Figure 3 illustrates a configuration of an elongate flexible PCB 302, having multiple conductive traces 304 laid along a length of the elongate flexible PCB 302, and multiple slits 306 cutting through the elongate flexible PCB 302 parallel to and between the conductive traces 304, and electric contacts 308 at one end of the conductive traces 304, the left end in Figure 3.

The configuration shown in Figure 3 is a configuration where the slits 306 split the elongate flexible PCB 302 into multiple separate strips attached to each other at one end, the left end of Figure 3. It is noted that a corresponding configuration, where the multiple separate strips are attached to each other at the other end, the right end of Figure 3, is also contemplated.

It is noted that a corresponding configuration, where the electric contacts 308 are at the other end, at the right of Figure 3, is also contemplated.

In some embodiments the elongate flexible PCB 302 may optionally include one or more of the slits 306 extending to both ends of the elongate flexible PCB 302 (not shown in Figure 3). In such a configuration (not shown in Figure 3) the slits 306 split the elongate flexible PCB 302 into multiple separate strips.

Proximal and distal ends of the elongate flexible PCB s 302 312 322 may or may not include contact pads or sensors.

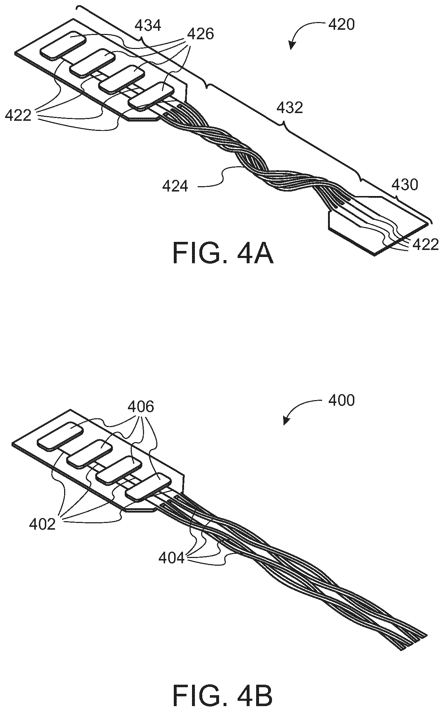

Reference is now made to Figure 4A, which is a simplified illustration of a PCB cable assembly according to an example embodiment.

Figure 4A shows an elongate flexible PCB forming a PCB cable assembly in which a group of traces are shown twisted.

Such an elongate flexible PCB provides a group of twisted traces which can potentially remove cross talk between signals carried via the traces, and/or remove cross talk between signals carried by neighboring groups of twisted traces.

In some embodiments, the elongate flexible PCB may optionally include shielding of the traces to shield from electromagnetic (EM) interference between signals carried via the groups of traces.

Figure 4A illustrates a configuration of an elongate flexible PCB 420, having multiple conductive traces 422 laid along a length of the elongate flexible PCB 420, and multiple slits cutting through the elongate flexible PCB 420 parallel to and between the conductive traces 422, and electric contacts 426 at one end of the conductive traces 422, the left end in Figure 4A.

The configuration shown in Figure 4A is a configuration where the electric contacts 426 are shown at the left end of Figure 4A. It is noted that a corresponding configuration, where the electric contacts 426 are at the other end, the right end of Figure 4A, is also contemplated. In some embodiments the electrical contacts 426 may be present at both ends of the conductive traces 422.

Figure 4A shows a non-limiting example of a specific number of strips 424 of twisted flexible PCB with traces.

In some embodiments a different number of twisted strips 424 may be used, for example 2, 3, 5, 6, 7, 8, 9, 10 strips twisted together, and even more.

In some embodiments a number of flexible PCB strips 420 may be twisted around each other, for example 2, 3, 4, 5, 6, 7, 8, 9, 10 flexible PCB strips, and even more, may be twisted around each other.

Reference is now made to Figure 4B, which is a simplified illustration of an elongate flexible PCB according to an example embodiment.

Figure 4B shows an elongate flexible PCB forming a PCB cable assembly, in which one or more pair(s) of traces are shown twisted.

Such an elongate flexible PCB provides twisted traces which can potentially remove cross talk between signals carried via the traces.

In some embodiments, the elongate flexible PCB may optionally include shielding of the traces to shield from electromagnetic (EM) interference between signals carried via the traces.

Figure 4B illustrates a configuration of an elongate flexible PCB 400, having multiple conductive traces 402 laid along a length of the elongate flexible PCB 400, and multiple slits cutting through the elongate flexible PCB 400 parallel to and between the conductive traces 402, and electric contacts 406 at one end of the conductive traces 402, the left end in Figure 4B.

The configuration shown in Figure 4B is a configuration where the slits split the elongate flexible PCB 400 into multiple separate strips attached to each other at one end, the left end of Figure 4B. It is noted that a corresponding configuration, where the multiple separate strips are attached to each other at the other end, the right end of Figure 4B, is also contemplated.

The multiple separate strips may optionally be twisted around each other.

Figure 4B shows a non-limiting example of two pairs 404 of twisted flexible PCB with traces.

In some embodiments a different number of twisted pairs 404 may be used, for example 1, 2, 3, 4, 5, 6, 7, 8, 9, 10 twisted pairs, and even more.

In some embodiments a different number of flexible PCB strips may be twisted around each other, for example 2, 3, 4, 5, 6, 7, 8, 9, 10 strips with traces, and even more, may be twisted around each other.

In some embodiments different combinations of different numbers of twisted strips may be combined in one flexible PCB. By way of a non-limiting example a twisted pair and a twisted triplet, or a twisted quadruplet, and other similar combinations.

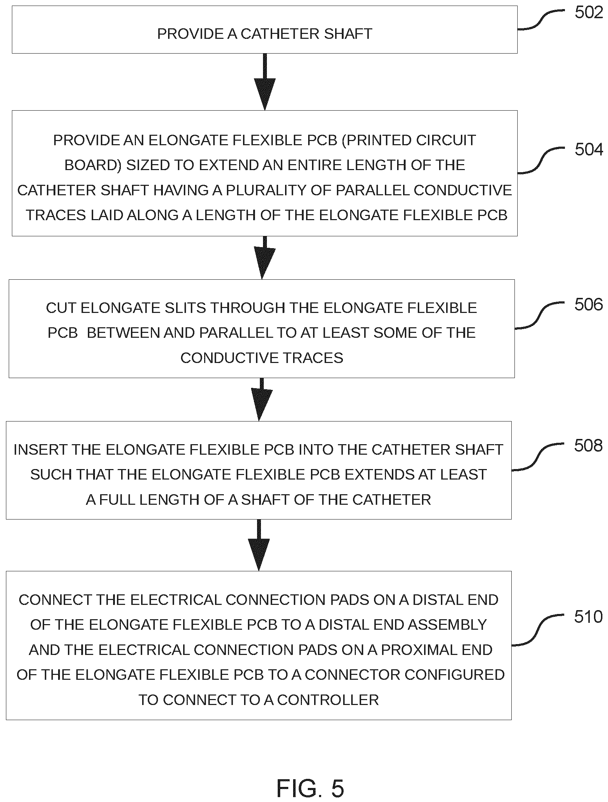

Reference is now made to Figure 5, which is a simplified flow chart illustration of a method of connecting a distal end assembly of a catheter to a connector at a proximal end of the catheter, according to an example embodiment.

The method exemplified in Figure 5 includes:
providing a catheter shaft (502);
providing an elongate flexible PCB (Printed Circuit Board) sized to extend an entire length of the catheter shaft having a plurality of parallel conductive traces laid along a length of the elongate flexible PCB (504;
cutting elongate slits through the elongate flexible PCB between and parallel to at least some of the conductive traces (506);
inserting the elongate flexible PCB into the catheter such that the elongate flexible PCB extends at least a full length of a shaft of the catheter (508); and
connecting the electrical connection pads on a distal end of the elongate flexible PCB to a distal end assembly and the electrical connection pads on a proximal end of the elongate flexible PCB to a connector configured to connect to a controller (510), wherein the controller is configured to communicate electrical signals between the controller and the distal end assembly (510).

In some embodiments elongate flexible PCB includes a plurality of electrical connection pads at ends of the conductive traces.

In some embodiments, at least some of the conductive traces are twisted about each other before the inserting the conductive cable into the catheter.

### SUMMARY

### Example 1

A catheter with a distal end assembly including a distal end assembly including an electrical connection array, an elongate flexible PCB (Printed Circuit Board) (200) being housed within a shaft of the catheter, the elongate flexible PCB extending substantially along a full length of the shaft and including a PCB distal portion (214) including a plurality of electrical connection pads (206) configured for establishing electrical connection with the electrical connection array of the distal sensing assembly, a PCB proximal portion (210) including a plurality of conductive traces (202) corresponding to the plurality of electric connection pads of the PCB distal portion, wherein the plurality of conductive traces is configured to be electrically connectable to an external electric connector, a PCB median portion (212) including a plurality of conductive traces (202, 304) laid longitudinally along more than 75% of a length of the elongate flexible PCB, the conductive traces continuing the conductive traces of the PCB proximal portion and electrically connected to the plurality of electrical pads of the PCB distal portion, and a plurality of slits (204, 306) cutting through the PCB median portion between the conductive traces and extending along the conductive traces.

### Example 2

The catheter according to Example 1 wherein the plurality of slits (204, 306) extends along more than 75% of the elongate flexible PCB.

### Example 3

The catheter according to any one of Examples 1-2, wherein at least two of the traces separated by a slit are twisted (404, 424) around each other.

### Example 4

The catheter according to any one of Examples 1-3, wherein all of the traces are twisted (424) along an elongate axis of the elongate flexible PCB.

### Example 5

The catheter according to any one of Examples 1-4 wherein the PCB median portion (212, 432) includes 10 or more conductive traces.

### Example 6

The catheter according to any one of Examples 1-5, wherein at least some of the slits (204, 306) extend all the way to an end of the flexible PCB.

### Example 7

The catheter according to any one of Examples 1-6, including a plurality of elongate flexible PCBs being housed within the shaft of the catheter.

### Example 8

A method of connecting a distal end assembly of a catheter to a connector at a proximal end of the catheter, the method including providing a catheter shaft (502), providing an elongate flexible PCB (Printed Circuit Board) sized to extend an entire length of the catheter shaft, having a plurality of parallel conductive traces laid along a length of the elongate flexible PCB (504), cutting elongate slits through the elongate flexible PCB between and parallel to at least some of the conductive traces (506), inserting the elongate flexible PCB into the catheter shaft such that the elongate flexible PCB extends at least a full length of the shaft of the catheter (508), and connecting the electrical connection pads on a distal end of the elongate flexible PCB to a distal end assembly and the electrical connection pads on a proximal end of the elongate flexible PCB to a connector configured to connect to a controller (510), wherein the controller is configured to communicate electrical signals between the controller and the distal end assembly.

### Example 9

The method according to Example 8 wherein the elongate flexible PCB includes a plurality of electrical connection pads at ends of the conductive traces

### Example 10

The method according to any one of Examples 8-9 wherein cutting slits through the elongate flexible PCB between and parallel to at least some of the conductive lines includes cutting slits between all of the parallel conductive lines.

### Example 11

The method according to any one of Examples 8-10 and further including rolling the flexible PCB to an elongate tube shape.

### Example 12

The method according to any one of Examples 8-11 and further including twisting a median portion of the elongate flexible PCB.

### Example 13

The method according to any one of Examples 8-12 and further including twisting at least some of the conductive traces about each other, thereby producing a twisted plurality of conductors.

### Example 14

The method according to any one of Examples 8-13 wherein cutting slits in the flexible PCB between at least some of the parallel conductive traces includes leaving at least one end of the flexible PCB united.

### Example 15

The method according to any one of Examples 8-14 wherein cutting slits in the flexible base between at least some of the parallel conductive lines includes leaving both ends of the flexible PCB united.

### Example 16

A group of conductors including a plurality of elongate flexible strips, each strip including a flexible PCB (200, 302, 400, 420), the flexible PCB including a PCB distal portion (214, 434) including a plurality of electrical connection pads (206, 406, 426) configured for establishing electrical connection with an electrical connection array of a distal sensing assembly, a PCB proximal portion (210, 430) including a plurality of conductive traces (202, 304, 402, 422) corresponding to the plurality of electric connection pads (206, 406, 426) of the PCB distal portion, wherein the plurality of conductive traces is configured to be electrically connectable to an external electric connector, a PCB median portion (212, 432) including a plurality of conductive traces (202, 304, 402, 422) laid longitudinally along more than 75% of a length of the elongate flexible PCB, the conductive traces continuing the conductive traces of the PCB proximal portion and electrically connected to the plurality of electrical pads of the PCB distal portion, and a plurality of slits (204, 306) cutting through the PCB median portion between the conductive traces and extending along the conductive traces, and wherein the PCB median portion includes at least some of the conductive traces twisted (404, 424) about each other.

### Example 17

The group according to Example 16 wherein the conductive traces are insulated from each other.

### Example 18

The group according to any one of Examples 16-17 wherein the PCB median portion includes insulation sheathing the conductive traces.

### Example 19

The group according to any one of Examples 16-18 wherein the flexible PCB includes electromagnetic (EM) shielding.

### Example 20

The group according to any one of Examples 16-19 wherein the group is sheathed by a catheter.

### Example 21

The group according to Example 20 wherein the group extends from a proximal end to a distal end of the catheter.

### Example 22

The group according to any one of Examples 20-21 wherein the group includes 10 or more conductive traces.

### Example 23

The group according to any one of Examples 16-22 wherein at least some of the flexible strips are twisted (404, 424) around each other.

### Example 24

The group according to any one of Examples 16-23 wherein the flexible strips are united at one end.

### Example 25

The group according to any one of Examples 16-23 wherein the flexible strips are attached to each other at one end.

### Example 26

The group according to any one of Examples 16-23 wherein the flexible strips are united at both ends.

### Example 27

The group according to any one of Examples 16-23 wherein the flexible strips are attached to each other at both ends.

### Example 28

The group according to any one of Examples 16-27 wherein at least one of the conductive lines is connected to a sensor.

As such, those skilled in the art to which the present invention pertains, can appreciate that while the present invention has been described in terms of preferred examples, the concept upon which this disclosure is based may readily be utilized as a basis for the designing of other structures, systems and processes for carrying out the several purposes of the present invention.

Also, it is to be understood that the phraseology and terminology employed herein are for the purpose of description and should not be regarded as limiting.

It should be noted that the words "comprising", "including" and "having" as used throughout the appended claims are to be interpreted to mean "including but not limited to". The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases, and disjunctively present in other cases.

It is important, therefore, that the scope of the invention is not construed as being limited by the illustrative examples set forth herein. Other variations are possible within the scope of the present invention as defined in the appended claims. Other combinations and sub-combinations of features, functions, elements and/or properties may be claimed through amendment of the present claims or presentation of new claims in this or a related application. Such amended or new claims, whether they are directed to different combinations or directed to the same combinations, whether different, broader, narrower or equal in scope to the original claims, are also regarded as included within the subject matter of the present description.

It is expected that during the life of a patent maturing from this application many relevant flexible PCB materials will be developed and the scope of the term flexible PCB is intended to include all such new technologies *a priori.*

The terms "comprising", "including", "having" and their conjugates mean "including but not limited to".

As used herein, the singular form "a", "an" and "the" include plural references unless the context clearly dictates otherwise.

The term "example" is used herein to mean "serving as an example, instance or illustration". Any embodiment described as an "example" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the disclosure may include a plurality of "optional" features unless such features conflict.

Throughout this application, various embodiments of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible sub-ranges as well as individual numerical values within that range, including minimum and maximum values of the range and fractional values in the range.

Unless otherwise indicated, numbers used herein and any number ranges based thereon are approximations within the accuracy of reasonable measurement and rounding errors as understood by persons skilled in the art.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination or as suitable in any other described embodiment of the disclosure. Certain features described in the context of various embodiments are not to be considered essential features of those embodiments, unless the embodiment is inoperative without those elements.

To the extent that section headings are used, they should not be construed as necessarily limiting.

## Claims

1. A catheter (12, 240) with a distal end assembly comprising:
a distal end assembly (10, 238) comprising an electrical connection array;
an elongate flexible PCB (Printed Circuit Board) (200, 234, 302, 400, 420) being housed within a shaft of the catheter, the elongate flexible PCB extending substantially along a full length of the shaft and comprising:
a PCB distal portion (214, 236, 434) comprising a plurality of electrical connection pads (206) configured for establishing electrical connection with the electrical connection array of the distal end assembly (10, 238);
a PCB proximal portion (210, 232) comprising a plurality of conductive traces (202, 304, 404, 424) corresponding to the plurality of electric connection pads (206, 426) of the PCB distal portion (214, 236, 434), wherein the plurality of conductive traces is configured to be electrically connectable to an external electric connector;
a PCB median portion (212, 432) comprising:
a plurality of conductive traces (202, 304, 404, 424) laid longitudinally along more than 75% of a length of the elongate flexible PCB, said conductive traces continuing the conductive traces of the PCB proximal portion and electrically connected to the plurality of electric connection pads of the PCB distal portion; and
a plurality of slits (204, 306) cutting through the PCB median portion between the conductive traces and extending along said conductive traces.

2. The catheter according to claim 1 wherein the plurality of slits extends along more than 75% of the elongate flexible PCB.

3. The catheter according to any one of claims 1-2, wherein at least two of the traces separated by a slit are twisted around each other.

4. The catheter according to any one of claims 1-3, wherein all of the traces are twisted along an elongate axis of the elongate flexible PCB.

5. The catheter according to any one of claims 1-4, wherein at least some of the slits extend all the way to an end of the elongate flexible PCB.

6. A method of connecting a distal end assembly (10, 238) of a catheter (12, 240) to a connector at a proximal end of the catheter, the method comprising:
providing a catheter shaft;
providing an elongate flexible PCB (Printed Circuit Board) (200, 234, 302, 400, 420) sized to extend an entire length of the catheter shaft, having a plurality of parallel conductive traces (202, 304, 404, 424) laid along a length of the elongate flexible PCB;
cutting elongate slits (204, 306) through the elongate flexible PCB between and parallel to at least some of the conductive traces;
inserting the elongate flexible PCB into the catheter shaft such that the elongate flexible PCB extends at least a full length of the shaft of the catheter; and
connecting electrical connection pads (206, 426) on a distal end of the elongate flexible PCB to a distal end assembly and electrical connection pads on a proximal end of the elongate flexible PCB to a connector configured to connect to a controller, wherein the controller is configured to communicate electrical signals between the controller and the distal end assembly.

7. The method according to claim 6 wherein cutting slits through the elongate flexible PCB between and parallel to at least some of the conductive traces comprises cutting slits between all of the parallel conductive traces.

8. The method according to any one of claims 6 and 7 and further comprising rolling the flexible PCB to an elongate tube shape.

9. The method according to any one of claims 6-8 and further comprising twisting a median portion of the elongate flexible PCB.

10. The method according to any one of claims 6-9 and further comprising twisting at least some of the conductive traces about each other, thereby producing a twisted plurality of conductors.

11. A group of conductors comprising a plurality of elongate flexible strips, each strip comprising:
a flexible PCB (200, 234, 302, 400, 420), the flexible PCB comprising:
a PCB distal portion (214, 236, 434) comprising a plurality of electrical connection pads (206) configured for establishing electrical connection with an electrical connection array of a distal sensing assembly;
a PCB proximal portion (210, 232) comprising a plurality of conductive traces (202, 304, 404, 424) corresponding to the plurality of electrical connection pads of the PCB distal portion, wherein the plurality of conductive traces is configured to be electrically connectable to an external electric connector;
a PCB median portion (212, 432) comprising:
a plurality of conductive traces (202, 304, 404, 424) laid longitudinally along more than 75% of a length of the elongate flexible PCB, said conductive traces continuing the conductive traces of the PCB proximal portion and electrically connected to the plurality of electrical pads of the PCB distal portion, and;
a plurality of slits (204, 306) cutting through the PCB median portion between the conductive traces and extending along said conductive traces; and
wherein the PCB median portion comprises at least some of the conductive traces twisted about each other.

12. The group according to claim 11 wherein the PCB median portion comprises insulation sheathing the conductive traces.

13. The group according to any one of claims 11 and 12 wherein the flexible PCB comprises electromagnetic (EM) shielding.

14. The group according to any one of claims 11-13 wherein the group is sheathed by a catheter (12, 240) and the group extends from a proximal end to a distal end of the catheter.

15. The group according to any one of claims 11-14 wherein at least some of the flexible strips are twisted around each other.
